# EUROPEAN PATENT APPLICATION

(11) **EP 3 028 693 A1**
(43) Date of publication of application: **08.06.2016**
(21) Application number: 14832892.5
(22) Date of filing: 24.07.2014
(51) Int. Cl.: A61K 9/28, A61K 31/436

(54) **STABLE SOLID IMMUNOSUPPRESSOR COMPOSITION**

(30) Priority: 02.08.2013 MX 2013008994
(71) Applicant: Laboratorio Raam de Sahuayo, S.a. de C.V., 59000 Sahuayo, Michoacán (MX)
(72) Inventor: AMEZCUA AMEZCUA, Federico, 59000 Sahuayo, Michoacán (MX)
(74) Representative: Balder IP Law, S.L.
(86) International application number: PCT/MX2014/000113
(87) International publication number: WO 2015/016694

(57) **Abstract**

The present invention consists in a stable solid pharmaceutical composition comprising 1) one or more immunosuppressant agents and/or pharmaceutically acceptable salts thereof; 2) a stabilizing mixture comprising i) vitamin E oil, ii) L-leucine, and iii) one or more poloxamers; and, 3) one or more pharmaceutically acceptable excipients, contained in at least one dosage unit.

## Description

### Field of the Invention

The present invention consists in a stable solid pharmaceutical composition comprising 1) one or more immunosuppressant agents and/or pharmaceutically acceptable salts thereof; 2) a stabilizing mixture consisting of i) vitamin E oil, ii) L-leucine, and iii) poloxamer; and, 3) one or more pharmaceutically acceptable excipients, contained in at least one dosage unit.

### Background

The immune system is a body's natural defense against infections, which by a number of different processes combats and destroys invading infectious agents that can cause harm to the body. Such agents can be viruses, bacteria, fungi, toxins, cancer cells among other more complex, which contain proteins known as antigens on its surface, same that are recognized and attacked by the immune system to control them until they are eliminated from the body.

For organ transplants, the same reaction of the immune system is carried out, which detects and attacks antigens of the new organ, causing rejection of that organ, so for transplantation it is required that antigens of donor and recipient be as similar as possible in order to reduce the likelihood of rejection; however, that probability is not excluded in a hundred percent; therefore, agents must be used to suppress the immune system.

Immunosuppressant agents have the ability to induce immunosuppression on the immune system via different mechanisms of action, such as: inhibition of transcription factors and proteins associated, like glucocorticoids exerting its immunosuppressant and anti-inflammatory action primarily by inhibiting the expression of the genes of interleukin-2 (IL-2) and other mediators; inhibitors of pyrimidine synthesis, such as leflunomide; inhibitors of TOR protein (a phosphatidylinositol-3-kinase required for activation and subsequent progression of LTs from the G1 phase to the S phase of the cell cycle, as the sirolimus or rapamycin; interference of nucleic acids, such as cyclophosphamide and antimalarial; antineoplastics as methotrexate; biological agents such as TNF-α antagonists (etanercept, infliximab, adalimumab), antagonists of IL-1 (anakinra), anti-CD20 antibodies (rituximab), among others.

Sirolimus or rapamycin, is a lipophilic macrolide obtained from fungus *Streptomyces hygroscopicus,* recently incorporated in clinical practice, which has many structural similarities to tacrolimus. FDA has approved it for prophylaxis and treatment of heart, lung, pancreas, kidney, and liver transplant rejection.

The mechanism of action of sirolimus consists in binding FKBP12, like tacrolimus, but unlike this, does not inhibit calcineurin activity. In fact, the Sirolimus-FKBP12 complex is a highly specific inhibitor of TOR protein, a phosphatidylinositol-3-kinase required for activation and subsequent synthesis of P70S6K, an enzyme that is critical in the activation of ribosomal protein S6. TOR inhibition prevents P34CDC2 kinase activity, which forms a complex with cyclin E, preventing the elimination of P27KLP, a negative regulatory factor of the cyclin-dependent kinases (CDKs), and of the binding protein of eukaryotic initiation factor (elF-4F), which is necessary for protein translation. As a result of these activities, sirolimus inhibits LT progression from the G1 phase to the S phase of the cell cycle.

The adverse effects described for sirolimus have been categorized and divided into metabolic, hematologic, dermatological, and related to the inhibition of growth factors. The main adverse effects are mixed dyslipidemia, pancytopenia and slight alterations in liver enzymes. These depend on the dose administered and can usually be controlled by reducing the dose. Interstitial pneumonia is a growing problem, which disappear when the medicine is withdrawn.

In addition, it has been reported that more than 20% of patients receiving combination therapy with sirolimus and cyclosporine A had tachycardia, hypotension, paresthesia, lassitude, fever, abdominal pain, diarrhea, pruritus, anemia, thrombocytopenia, peripheral edema, arthralgia and weight gain. At humoral level, hypercholesterolemia, hyperglycemia, hypocalcemia, increased liver enzymes (GOT, GPT), azotemia, hyperkalemia, the above secondary to the synergistic effect existing between both drugs, which consists in a 1000-fold greater inhibitory effect than each one separately.

In the state of the art, there are compositions which comprise: a solid dosage unit of rapamycin comprising a core and a sugar coating, characterized because it comprises rapamycin (sirolimus), one or more surface-modifier agents (poloxamer), and one or more sugars such as sucrose (MX219798), a solid dose unit of sirolimus with sugar-coated core, and binders (MX227633), tablets or capsules comprising sirolimus, a vehicle containing povidone K30, poloxamer 188 and the vitamin E polietanediol succinate (CN1939302), an oral composition in a tablet comprised by a monolithic matrix with sirolimus (with a D90 less than 10 µm, sucrose, lactose monohydrate, microcrystalline cellulose, crospovidone, poloxamer 188, glyceryl monooleate, vitamin E acetate, citric acid monohydrate, povidone K30, and talc (WO2011135580), among others that refer to compositions of sirolimus in the form of dispersible tablets, solutions, ophthalmic solutions, liposomes, suspensions, injectable, gel, ointments, suppositories, micro particles, coated devices, tablets, capsules, coated cores, or redispersible powder.

### Object of the Invention

The present invention relates to the development of new stable compositions of one or more immunosuppressive agents useful for the prevention and/or control the rejection of transplanted organs, the characteristic stability of the present composition is achieved through the synergistic combination of two preservatives combined with one or more pharmaceutically acceptable excipients. In addition, manufacturing methods of the composition are disclosed, so that it has the characteristic stability and a particular dissolution profile.

### Brief Description of the Drawings

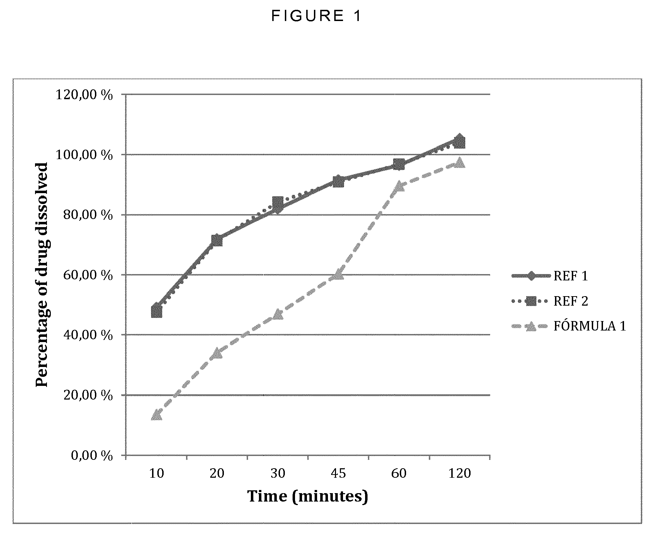

FIGURE 1. Dissolution profile of a sirolimus composition.

### Detailed Description of the Invention

The present invention consists in a stable solid pharmaceutical composition comprising: 1) one or more immunosuppressant agents and/or pharmaceutically acceptable salts thereof; 2) a stabilizing mixture comprising i) vitamin E oil, ii) L-leucine, and iii) one or more poloxamers; and, 3) one or more pharmaceutically acceptable excipients, contained in at least one dosage unit.

The key characteristic of the present invention is the stability achieved with the combination of two preservatives, which are vitamin E oil and L-leucine. The Vitamin E oil is a fat soluble vitamin which is used in the prior art as an antioxidant, stabilizing agent or preservative, however, due to this invention, a synergistic combination was developed in its function as an antioxidant agent consisting of mixing said excipient with L-leucine, an amino acid useful as stabilizing agent that surprisingly and unexpectedly increases the stability of the composition.

The present composition is further characterized by being substantially free of sugars such as sucrose, dextrose, fructose, mannitol, xylitol and sorbitol, as hydrolysis of sugars significantly affects the stability of compositions containing any protein and/or amino acid, and since the present composition contains L-leucine as a stabilizing agent, the presence of some sugar would decrease the effectiveness in terms of stability conservation.

The present composition is characterized by containing from 0.01 to 20 percent by total weight of the composition of one or more immunosuppressant agents, and/or their pharmaceutically acceptable salts, and/or polymorphs, and/or prodrugs selected from the following group: glucocorticoids, leflunomide, sirolimus, cyclophosphamide, antimalarials, methotrexate, etanercept, infliximab, adalimumab, anakinra, rituximab, and/or combinations thereof. Preferably, the immunosuppressant agent is sirolimus and/or pharmaceutically acceptable salts thereof, and/or polymorphs and/or prodrugs.

The stabilizing mixture is characterized by containing from 0.1 to 10 percent by total weight of the composition of vitamin E oil and/or pharmaceutically acceptable salts thereof, 0.1 to 10 percent of L-leucine by total weight of the composition, and 0.1 to 15.0 percent of one or more poloxamers by total weight of the composition.

Besides one or more immunosuppressive agents and the stabilizing mixture, the composition may contain from 20 to 99.9 percent of one or more pharmaceutically acceptable excipients selected from the following group: microcrystalline cellulose, magnesium stearate, crospovidone, purified water, polyvinylpyrrolidone, povidone, ethanol, lactose, hydroxypropyl methylcellulose, polyethylene glycol, propylene glycol, polypropylene glycol, stearic acid, starch, pregelatinized starch, among others, by total weight of the composition.

The compositions developed as part of the present invention are shown below by way of illustrative examples, but not limitative.

### Examples

**Example 1: Solid composition of sirolimus without stabilizing mixture.**

| Amount (mg) | Component |
|---|---|
| 1.00 | Sirolimus 30 |
| 4.00 | Crospovidone |
| 42.00 | Microcrystalline cellulose |
| 1.00 | Magnesium stearate |
| 42.00 mg | Lactose |
| 10.00 mg | PVP |
| q. s. | Absolute ethanol |
| q. s. | Purified water |

**Example 2: Solid composition of sirolimus with vitamin E oil.**

| Amount (mg) | Component |
|---|---|
| 1.00 | Sirolimus |
| 2.00 | Vitamin E oil |
| 6.00 | Crospovidone |
| 45.00 | Microcrystalline cellulose |
| 40.00 | Pregelatinized starch |
| 2.00 | Stearic acid |
| 45.00 | Lactose |
| 4.00 | PVP |
| q. s. | Absolute ethanol |

**Example 3: Solid composition of sirolimus with vitamin E oil and L-leucine.**

| Amount (mg) | Component |
|---|---|
| 1.00 | Sirolimus |
| 2.00 | Vitamin E oil |
| 2.00 | L-leucine |
| 6.00 | Crospovidone |
| 45.00 | Microcrystalline cellulose |
| 40.00 | Pregelatinized starch |
| 2.00 | Stearic acid |
| 45.00 | Lactose |
| 4.00 | PVP |
| q. s. | Absolute ethanol |

**Example 4: Solid composition of sirolimus with vitamin E oil, L-leucine and poloxamer.**

| Amount (mg) | Component |
|---|---|
| 1.00 | Sirolimus |
| 2.00 | Vitamin E oil |
| 2.00 | L-leucine |
| 8.00 | poloxamer |
| 6.00 | Crospovidone |
| 75.00 | Microcrystalline cellulose |
| 30.00 | Pregelatinized starch |
| 2.00 | Stearic acid |
| 45.00 | Lactose |
| 4.00 | PVP |
| 2.00 | Polyethylene glycol |
| q. s. | Absolute ethanol |

To determine the stability of the proposed compositions, accelerated stability testing were performed for different compositions containing various excipients and stabilizings, preservatives or antioxidants, obtaining superior stability in the composition of Example 4 compared to the other compositions of

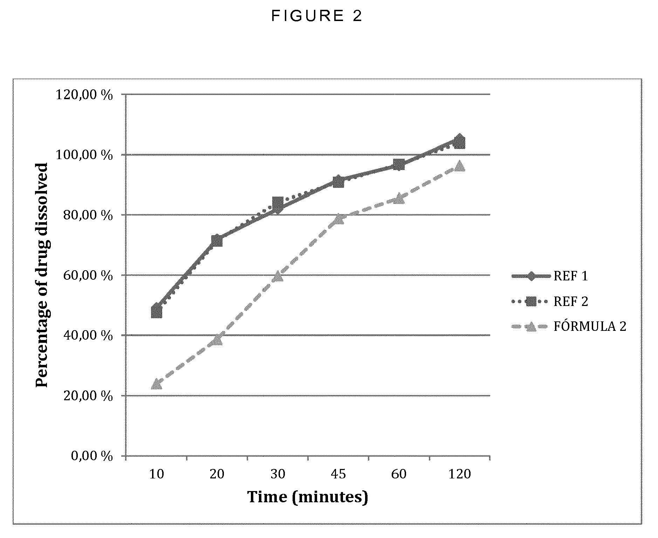

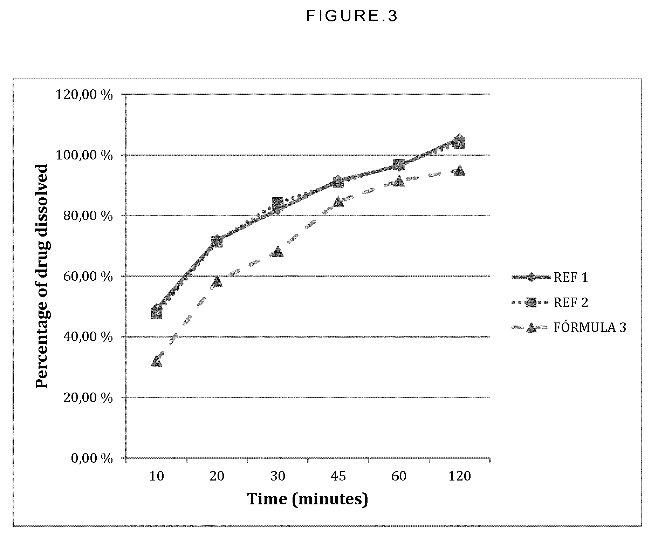

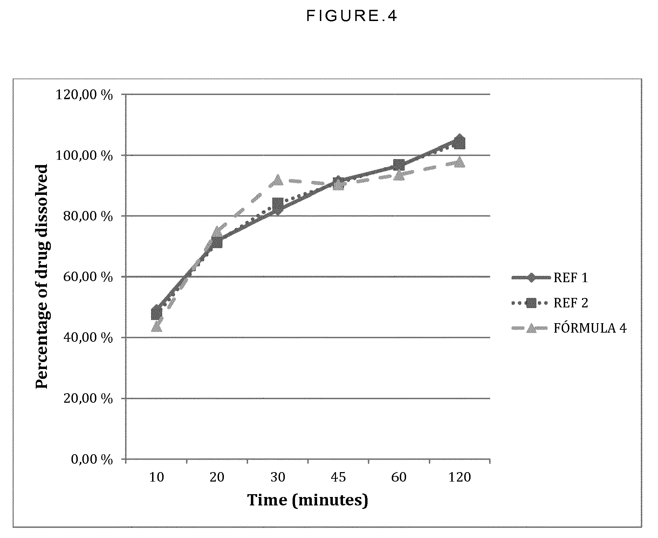

According to dissolution testing performed on the various formulations of Examples 1 to 4, based on the addition of the components of the stabilizing mixture, the percentage of dissolved drug improves in comparison with a reference product (REF 1 and REF 2), i. e., equivalence to the reference drug is achieved in the dissolution test. In Example 1 (FORMULA 1), which does not contain the stabilizing mixture, a completely different profile was obtained in comparison with the reference (REF 1 and REF 2), and does not reach 100% of dissolved drug (FIGURE 1); In Example 2 (FORMULA 2) containing only vitamin E oil as a stabilizing agent, it has a dissolution profile with significant differences compared to the reference drug (FIGURE 2); in Example 3, the dissolution profile slightly approaches to the behavior of the reference product without reaching 100 percent of dissolved drug, this composition is characterized by containing vitamin E oil and L-leucine (FIGURE 3); finally, FORMULA 4 of Example 4 containing the stabilizing mixture with vitamin E oil, L-leucine and poloxamer shows a dissolution profile equivalent or very similar to that obtained with the reference drug (FIGURE 4). In short, in addition to stability, the composition is characterized by a dissolution profile equivalent to the reference drug, thereby complying with the legislation on generic drugs.

The present composition has, without limitation, the following advantages:
1. Stable immunosuppressant composition.
2. Composition free of sugars as sucrose, dextrose, fructose, mannitol, xylitol, and sorbitol.
3. Composition with a suitable dissolution profile.
4. The composition provides the possibility of treatments using two or more drugs, ensuring its stability.
5. Equivalent or improved dissolution profile compared to the reference drug.

## Claims

1. A stable solid pharmaceutical composition comprising: 1) one or more immunosuppressant agents and/or pharmaceutically acceptable salts thereof; 2) a stabilizing mixture consisting of i) vitamin E oil, ii) L-leucine, and iii) one or more poloxamers; and, 3) one or more pharmaceutically acceptable excipients, contained in at least one dosage unit.

2. A pharmaceutical composition according to claim 1, **characterized by** containing from 0.01 to 20 percent of one or more immunosuppressant agents and/or pharmaceutically acceptable salts thereof, and/or polymorphs and/or prodrugs by total weight of the composition.

3. A pharmaceutical composition according to claims 1 and 2 wherein the immunosuppressive agent is selected from the following group: glucocorticoids, leflunomide, sirolimus, cyclophosphamide, antimalarials, methotrexate, etanercept, infliximab, adalimumab, anakinra, rituximab, and/or combinations thereof; and/or pharmaceutically acceptable salts thereof.

4. A pharmaceutical composition according to claims 1 and 2, wherein the immunosuppressant agent is sirolimus, and/or its pharmaceutically acceptable salts and/or polymorphs, and/or prodrugs thereof.

5. A pharmaceutical composition according to claim 1, wherein the stabilizing mixture contains from 0.1 to 10 percent of vitamin E oil by total weight of the composition and/or pharmaceutically acceptable salts thereof; from 0.1 to 10 percent of L-leucine by total weight of the composition, and 0.1 to 15.0 percent of one or more poloxamers by total weight of the composition.

6. A pharmaceutical composition according to claim 1, wherein it contains from 20 to 99.9 percent of one or more pharmaceutically acceptable excipients selected from the following group: microcrystalline cellulose, magnesium stearate, crospovidone, purified water, polyvinylpyrrolidone, povidone, ethanol, lactose, hydroxypropyl methylcellulose, polyethylene glycol, propylene glycol, polypropylene glycol, stearic acid, starch, pregelatinized starch, among others, by total weight of the composition.

7. A composition according to claims 1 to 6, wherein the dosage unit is prepared in form of tablet, and/or coated tablet, and/or controlled-release tablet, and/or granules, and/or soft gelatin capsule, and/or hard gelatin capsule, and/or pellets, and/or micro tablets.

8. Use of the composition according to claims 1 to 7 for the prevention and/or treatment and/or control of organ transplant rejection.
